# EUROPEAN PATENT APPLICATION

(11) **EP 4 585 638 A1**
(43) Date of publication of application: **16.07.2025**
(21) Application number: 22957721.8
(22) Date of filing: 08.09.2022
(51) Int. Cl.: C08J 11/10, C08J 11/04, C08G 18/32, C08L 75/04

(54) **SELF-CATALYTIC DIRECTIONAL DEGRADATION METHOD FOR POLYURETHANE MATERIAL, POLYOL PREPARED THEREBY, AND POLYURETHANE MATERIAL**

(71) Applicant: Swancor Innovation & Incubation Co., Ltd., Nantou City, Nantou County 540028 (TW)
(72) Inventor: CHEN, Yao-Huang, Taichung City (TW); CHEN, Chun-An, Dali (TW); LIN, Tong-Yan, Caotun Township, Nantou County (TW); HSU, Chih-Kai, Caotun Township, Nantou County (TW)
(74) Representative: Ter Meer Steinmeister & Partner
(86) International application number: PCT/CN2022/117759
(87) International publication number: WO 2024/050749

(57) **Abstract**

Provided in the present invention are a self-catalytic directional degradation method for a polyurethane material, a polyol prepared thereby and a polyurethane material. The self-catalytic directional degradation method for a polyurethane material comprises: carrying out a crushing step, a mixing step, a degradation step and a high-temperature decompression step. The crushing step comprises forming polyurethane particles. The mixing step comprises mixing the polyurethane particles with an alcohol amine compound to form a degradation system, wherein the alcohol amine compound has a structure as shown in formula (I) or formula (II), the symbols in formula (I) and formula (II) being as defined in the specification. The degradation step comprises degrading a degradation system so as to obtain an intermediate product. The high-temperature decompression step comprises removing the alcohol amine compound from the intermediate product, so as to obtain a polyol. Therefore, the alcohol amine compound is used for heating and degrading the polyurethane material, and the obtained product polyol can be applied to the synthesis of the polyurethane material, thereby achieving the recovery cycle purpose.

## Description

### Technical Field

The present disclosure relates to a method for degradation of polyurethane material. More particularly, the present disclosure relates to a method for self-catalytic directional degradation of polyurethane material, a polyol prepared thereof and a polyurethane material.

### Description of Related Art

With the increasing global annual production and consumption of polyurethane materials, the amount of waste polyurethane materials is also continuously rising. Although the polyurethane materials can naturally degrade in the environment through sunlight and moisture, the degradation rate thereof is slow and lengthy, which still causes harms to the environment. Furthermore, the key raw materials for the polyurethane materials mostly come from petrochemical sources, making it impossible to recycle the waste polyurethane materials, and inevitably leading to excessive waste of the petrochemical resources.

Current methods for recycling and reusing the waste polyurethane materials include energy recycling method, physical recycling method, or chemical recycling method. Energy recycling method involves directly incinerating the waste polyurethane materials and recycling heat energy, but this method is only a method for transition that does not actively improve the overconsumption of petrochemical resources. However, physical recycling method involves separating the waste polyurethane materials into thermoplastic polyurethane and thermosetting polyurethane. Thermoplastic polyurethane can be reprocessed and reused through thermal treatment (hot press bonding, extrusion molding), while thermosetting polyurethane is crushed to make the material into fine particles, which can be used as fillers for new polyurethane materials or other materials. Additionally, chemical recycling method involves degrading the waste polyurethane materials into polyol resins through methods such as mild cracking method, pyrolysis method, amine degradation method, alkaline degradation method, hydrolysis method, and alcoholysis method for recycling and reuse.

In this regard, how to find a suitable degradation solutions and provides a low-energy, fast-degradation method for the polyurethane materials to enable the recycling and reuse of the polyurethane materials, has become a goal for the related industry.

### SUMMARY

One purpose of the present disclosure is to provide a method for self-catalytic directional degradation of polyurethane material and the polyol produced therefrom, wherein an alcoholamine compound is used to heat and decompose the polyurethane material, and the resulting polyol product includes a polyol with a urea structure.

Another purpose of the present disclosure is to provide a polyurethane material, wherein the polyol product obtained after degrading the polyurethane material is applied to the synthesis of polyurethane materials, achieving the goal of recycling and reuse.

One embodiment of the present disclosure provides a method for self-catalytic directional degradation of polyurethane material, which includes performing a crushing step, performing a mixing step, performing a degradation step, and performing a heating and pressure reduction step. In the crushing step, the polyurethane material is crushed to form several polyurethane particles. In a mixing step, the several polyurethane particles are mixed with an alcoholamine compound to form a degradation system, and the alcoholamine compound has a structure represented by formula (I) or formula (II): 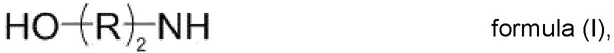 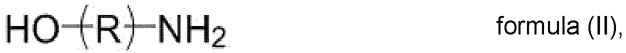 wherein R is an alkylene group having 2 to 6 carbon atoms or a structural isomer thereof, or a phenyl group or a derivative thereof. In the degradation step, the degradation system is degraded at a reaction temperature to obtain an intermediate product. In the heating and pressure reduction step, the intermediate product is under the reaction temperature and a reaction pressure to remove the alcoholamine compound, so as to obtain a polyol.

According to the method for self-catalytic directional degradation of polyurethane material of the aforementioned embodiment, wherein the polyurethane material is obtained through a polymerization reaction, and a monomer for the polymerization reaction can include an isocyanate component and an isocyanate-reactive component.

According to the method for self-catalytic directional degradation of polyurethane material of the aforementioned embodiment, wherein the isocyanate component can include aromatic diisocyanate, aliphatic diisocyanate, an aromatic diisocyanate derivative, an aliphatic diisocyanate derivative, polymethylene polyphenyl isocyanate, or a mixture thereof. Furthermore, the isocyanate-reactive component can include a polyester polyol, a polyether polyol, a small-molecule multifunctional compound, or a mixture thereof.

According to the method for self-catalytic directional degradation of polyurethane material of the aforementioned embodiment, wherein a particle diameter of each of the several polyurethane particles can be 2 mm to 5 mm.

According to the method for self-catalytic directional degradation of polyurethane material of the aforementioned embodiment, wherein a mass ratio of the several polyurethane particles to the alcoholamine compound can be 1:0.9 to 1:3.

According to the method for self-catalytic directional degradation of polyurethane material of the aforementioned embodiment, wherein the reaction temperature can be 110°C to 160°C.

According to the method for self-catalytic directional degradation of polyurethane material of the aforementioned embodiment, wherein in the degradation step, the degradation system is maintained at the reaction temperature for a degradation time, and the degradation time can be 30 minutes to 6 hours.

According to the method for self-catalytic directional degradation of polyurethane material of the aforementioned embodiment, wherein the reaction pressure can be 1 mbar to 1000 mbar.

According to the method for self-catalytic directional degradation of polyurethane material of the aforementioned embodiment, wherein a liquefaction ratio of the polyurethane material after degradation can be 100%.

Another embodiment of the present disclosure provides a polyol, which is obtained by the method for self-catalytic directional degradation of polyurethane material of the aforementioned embodiment.

According to the polyol of the aforementioned embodiment, the polyol has a structure represented by formula (III):

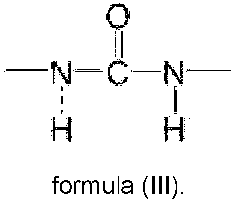

One another embodiment of the present disclosure provides a polyurethane material, which is obtained by the polyol of the aforementioned embodiment reacting with isocyanate.

Therefore, the method for self-catalytic directional degradation of polyurethane material according to the present disclosure primarily selects the alcoholamine compound with an amino group carrying an active hydrogen to heat and dissolve the polyurethane material, resulting in a polyol. No purification or rinsing steps are required, and only filtration and drying steps are needed to use the polyol as part of the polyurethane material, allowing the polyurethane material to be recycled and reused, which makes the material as an environmentally friendly material.

### BRIEF DESCRIPTION OF THE DRAWINGS

In order to make the above and other objects, features, advantages and examples of the present disclosure more obvious and understandable, the accompanying drawings are described as follows:
Fig. 1 shows a step flowchart of a method for self-catalytic directional degradation of polyurethane material according to an embodiment of the present disclosure.
Fig. 2 shows an infrared spectrum of the polyol after degradation.
Fig. 3 shows an infrared spectrum of the polyol after being dissolved in water and dried.

### [DENOTATION OF MAIN REFERENCE NUMERALS]

100: method for self-catalytic directional degradation of polyurethane material
110, 120, 130, 140: step

### DETAILED DESCRIPTION

The present disclosure will be further exemplified by the following specific embodiments. However, the embodiments can be applied to various inventive concepts and can be embodied in various specific ranges. The specific embodiments are only for the purposes of description, and are not limited to these practical details thereof.

In the present disclosure, the compound structure can be represented by a skeleton formula, and the representation can omit carbon atoms, hydrogen atoms and carbon-hydrogen bonds. If the functional groups are clearly identified in a structural formula, the identified structural formula should be followed.

In the present disclosure, in order to keep conciseness and smoothness, "the alcoholamine compound has a structure represented by formula (I)" can be described as the alcoholamine compound represented by formula (I) or the alcoholamine compound (I) in some cases, and the other compounds or groups can be described in the same manner.

### <Method for Self-catalytic Directional Degradation of Polyurethane Material>

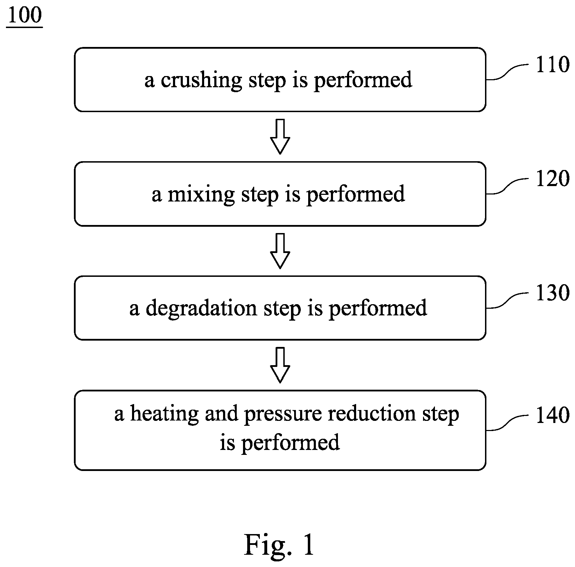

Referring to Fig. 1, Fig. 1 shows a step flowchart of a method for self-catalytic directional degradation of polyurethane material 100 according to an embodiment of the present disclosure. In Fig. 1, the method for self-catalytic directional degradation of polyurethane material 100 includes Step 110, Step 120, Step 130, and Step 140.

Step 110 is a crushing step, wherein a polyurethane material is crushed to form several polyurethane particles, and a particle diameter of each of the polyurethane particles can be 2 mm to 5 mm.

Specifically, the polyurethane material of the present disclosure is obtained through a polymerization reaction, and a monomer for the polymerization reaction includes an isocyanate component and an isocyanate-reactive component. The isocyanate component can include aromatic diisocyanate, aliphatic diisocyanate, an aromatic diisocyanate derivative, an aliphatic diisocyanate derivative, polymethylene polyphenyl isocyanate, or a mixture thereof. For example, the aromatic diisocyanate can be, but is not limited to, toluene diisocyanate (TDI), diphenylmethane diisocyanate (MDI), naphthalene diisocyanate (NDI), p-phenylene diisocyanate (PPDI), benzylidene diisocyanate (XDI), dimethyl biphenyl diisocyanate (TODI), dimethyl diphenylmethane diisocyanate (DMMDI), or a mixture of one or more of the aforementioned compounds; the aliphatic diisocyanate can be, but is not limited to, isophorone diisocyanate (IPDI), hexamethylene diisocyanate (HDI), dicyclohexylmethane diisocyanate (H₁₂MDI), 1,4-cyclohexane diisocyanate (CHDI), trimethyl-1,6-hexamethylene diisocyanate (TMHDI), methyl cyclohexyl diisocyanate (HTDI), or a mixture of one or more of the aforementioned compounds; the aromatic diisocyanate derivative can be, but is not limited to, toluene diisocyanate dimer (TDI-dimer), toluene diisocyanate trimer (TDI-trimer), or a mixture thereof; the aliphatic diisocyanate derivative can be, but is not limited to, hexamethylene diisocyanate dimer (HDI-dimer), hexamethylene diisocyanate trimer (HDI-trimer), hexamethylene diisocyanate biuret (HDI Biuret), isophorone diisocyanate trimer (IPDI-trimer), or a mixture of one or more of the aforementioned compounds.

Furthermore, the isocyanate-reactive component can include a polyester polyol, a polyether polyol, a small-molecule multifunctional compound, or a mixture thereof. For example, the polyester polyol can be, but is not limited to, an adipic acid-based polyester polyol, an aromatic polyester polyol, a polycaprolactone diol, a polycarbonate diol, or a mixture of one or more of the aforementioned compounds; the polyether polyol can be, but is not limited to, a poly(propylene oxide) polyol, a poly(ethylene oxide) polyol, a poly(tetrahydrofuran) polyol, or a mixture of one or more of the aforementioned compounds; the small-molecule multifunctional compound can be, but is not limited to, a diol, a polyol, an alcoholamine, a diamine compound, or a mixture of one or more of the aforementioned compounds.

Specifically, the diol can be, but is not limited to, ethylene glycol, 1,4-butanediol, diethylene glycol, triethylene glycol, 1,2-propylene glycol, neopentyl glycol, methylpropyl glycol, 1,6-hexanediol, 1,3-propanediol, dipropylene glycol, tripropylene glycol, butyl ethyl propylene glycol, diethyl pentanediol, 3-methyl-1,5-pentanediol, 1,3-butanediol, 1,2-butanediol, 2,3-butanediol, trimethylpentanediol, cyclohexanediol, or 1,4-dihydroxy methylcyclohexane; the polyol can be, but is not limited to, trimethylolpropane, glycerol, trimethylol ethane, 1,2,6-hexanetriol, trimethylol ethyl isocyanurate, pentaerythritol, xylitol, or sorbitol; the alcoholamine can be, but is not limited to, triethanolamine, diethanolamine, triisopropanolamine, methyl diethanolamine, bis-hydroxyisopropylbenzene, bis-hydroxyisopropyl-p-toluidine, dihydroxyethylphenylamine, dihydroxyethyl-p-toluidine, or dihydroxyethyl-m-xylenediamine; the diamine compound can be, but is not limited to, 3,3'-dichloro-4,4'-diphenylmethanediamine, 3,5-dimethylthio-toluenediamine, 3,5-diethyl-toluenediamine, 4,4'-methylene-bis(3-chloro-2,6-diethylphenylamine), 4,4'-methylene-bis(2,6-diethylphenylamine), 4,4'-methylene-bis(2,6-diisopropylphenylamine), 4,4'-methylene-bis(2-isopropyl-6-methylphenylamine), 4,4'-methylene-bis(2-isopropyl-6-diethylphenylamine), 4,4'-methylene-bis(2-ethylphenylamine), toluenediamine, 4,4'-diaminodiphenylmethane, isophorone diamine, diamino dicyclohexylmethane, trimethylhexamethylenediamine, or dimethyl diaminodicyclohexylmethane.

Step 120 is a mixing step, wherein the several polyurethane particles are mixed with an alcoholamine compound to form a degradation system, and the alcoholamine compound has a structure represented by formula (I) or formula (II): 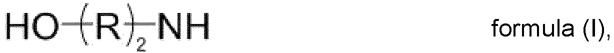 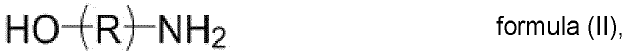 wherein R is an alkylene group having 2 to 6 carbon atoms or a structural isomer thereof, or a phenyl group or a derivative thereof. Specifically, the present disclosure uses the alcoholamine compound, which includes active hydrogen in the amine group, as the degradation solution. The degradation solution is a single-component system, and no solvents or additives are added. A mass ratio of the several polyurethane particles to the alcoholamine compound can be 1:0.9 to 1:3.

Step 130 is a degradation step, wherein the degradation system is under degradation at a reaction temperature to obtain an intermediate product, and the reaction temperature can be 110°C to 160°C. Specifically, the degradation system can be maintained at the reaction temperature for a degradation time, which can be 30 minutes to 6 hours. A liquefaction ratio of the polyurethane material after degradation can be 100%.

Step 140 is a heating and pressure reduction step, wherein the intermediate product is under the reaction temperature and a reaction pressure to remove the alcoholamine compound, so as to obtain a polyol. The reaction pressure can be 1 mbar to 1000 mbar. Specifically, because the alcoholamine compound has a low boiling point, it can easily be distilled and separated under low-pressure conditions. Therefore, the intermediate product can be under a temperature of 110°C to 160°C and a pressure of 1 mbar to 1000 mbar to remove the excess alcoholamine compound, and after cooling, the polyol product can be obtained. The alcoholamine compound removed by the heating and pressure reduction step can be reused as a degradation solution for polyurethane material.

Therefore, the method for self-catalytic directional degradation of polyurethane material 100 according to the present disclosure utilizes the alcoholamine compound, as shown in formula (I) or formula (II), as the degradation solution. Due to the structure thereof including both an amine group and an alcohol group with active hydrogen, it can selectively open the carbamate bonds in the polyurethane material structure, further control the reaction temperature, and prevent the urethane group in the polyurethane material from degrading and opening to avoid the formation of aromatic polyamines. It allows the final degraded polyol product to be directly reused in the synthesis of the polyurethane material without further purification.

The present disclosure further provides a polyol obtained by the aforementioned method for self-catalytic directional degradation of polyurethane material 100. Specifically, through the use of the alcoholamine compound for heating and degradation of polyurethane material, a polyol including a urea structure can be obtained, which can have a structure represented by formula (III):

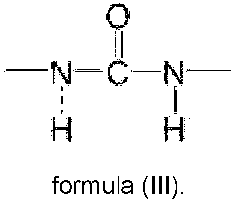

Furthermore, the polyol obtained by the method for self-catalytic directional degradation of polyurethane material 100 according to the present disclosure can be a polyol mixture rather than a single polyol. At least a portion of the polyol can be the polyol including a urea structure, and in certain embodiments, the polyol including the urea structure can account for 1% to 100% of the polyol mixture obtained after degradation, for example, for 10%, 30%, 50%, 70%, 90%, or 100%.

### <Polyurethane Material>

The present disclosure further provides a polyurethane material, which is obtained by the aforementioned polyol reacting with isocyanate. For details on the isocyanate, reference can be made to the types of the isocyanate component mentioned earlier, and further details thereof are not given again herein. Specifically, the polyol product obtained through the method for self-catalytic directional degradation of polyurethane material 100 according to the present disclosure can be used as a polyol in the polyurethane material without any purification or rinsing steps. It only requires filtration and drying before being applied. Additionally, the functionality of the polyol product can be determined by the hydroxyl value, pre-polymerization reaction, and viscosity changes thereof. It can be used in various polyurethane materials, such as polyurethane rigid foam, polyurethane adhesives, or thermoplastic polyurethane, achieving the goal of recycling.

The present disclosure will be further exemplified by the following specific embodiments so as to facilitate utilizing and practicing the present disclosure completely by the people skilled in the art without over-interpreting and over-experimenting. However, the readers should understand that the present disclosure should not be limited to these practical details thereof, that is, these practical details are used to describe how to implement the materials and methods of the present disclosure and are not necessary.

### <Example>

### <Degradation of Polyurethane Material>

Example 1: 100 grams of polyurethane resin particles (synthetic thermosetting PU) are placed in a 500 mL glass reaction flask, and 200 grams of diethanolamine are added. The reaction temperature is controlled at 150°C, and the reaction is carried out for 1.5 hours. Once the solution becomes clear and transparent, excess diethanolamine is distilled off to obtain the melted polyether polyol. The polyether polyol prepared in Example 1 has an OH value of 600, a viscosity of 200 cps, and a liquefaction ratio of polyurethane of 100%.

Example 2: 100 grams of polyurethane resin particles (waste polyurethane foam material) are placed in a 500 mL glass reaction flask, and 200 grams of isopropanolamine are added. The reaction temperature is controlled at 130°C, and the reaction is carried out for 4 hours. Once the solution becomes clear and transparent, excess isopropanolamine is distilled off to obtain the melted polyether polyol. The polyether polyol prepared in Example 2 has an OH value of 800, a viscosity of 110 cps, and a liquefaction ratio of polyurethane of 100%.

Example 3: 100 grams of polyurethane resin particles (waste polyurethane foam material) are placed in a 500 mL glass reaction flask, and 200 grams of diethanolamine are added. The reaction temperature is controlled at 110°C, and the reaction is carried out for 6 hours. Once the solution becomes clear and transparent, excess diethanolamine is distilled off to obtain the melted polyether polyol. The polyether polyol prepared in Example 3 has an OH value of 700, a viscosity of 130 cps, and a liquefaction ratio of polyurethane of 100%.

Example 4: 100 grams of polyurethane resin particles (waste polyurethane foam material) are placed in a 500 mL glass reaction flask, and 200 grams of diisopropanolamine are added. The reaction temperature is controlled at 160°C, and the reaction is carried out for 2 hours. Once the solution becomes clear and transparent, excess diisopropanolamine is distilled off to obtain the melted polyether polyol. The polyether polyol prepared in Example 4 has an OH value of 650, a viscosity of 200 cps, and a liquefaction ratio of polyurethane of 100%.

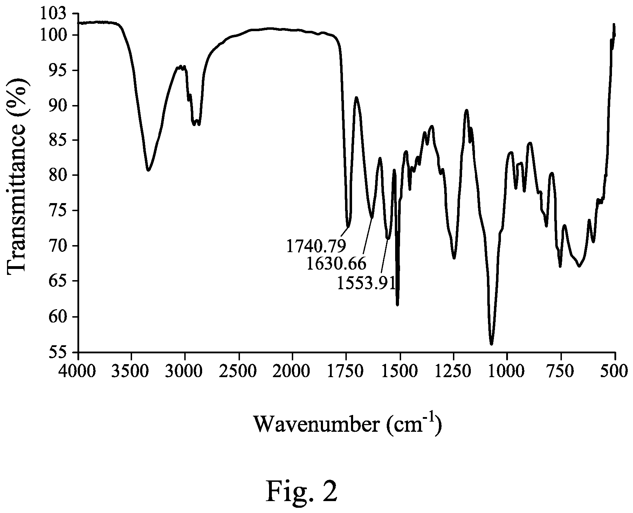

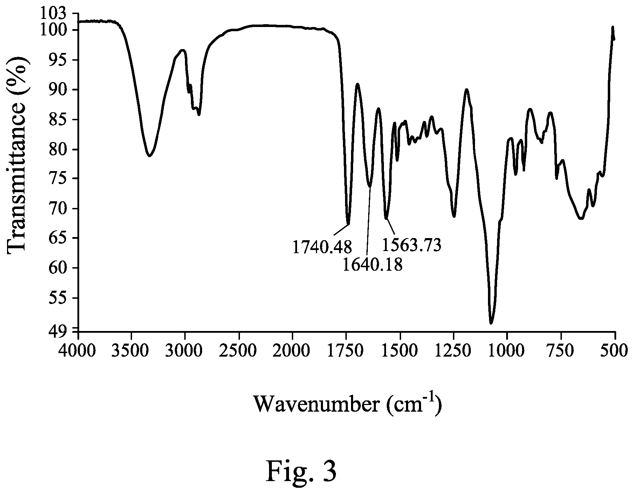

Referring to Fig. 2 and Fig. 3, Fig. 2 shows an infrared spectrum of the polyol after degradation, and Fig. 3 shows an infrared spectrum of the polyol after being dissolved in water and dried. From the results of Fig. 2 and Fig. 3, it can be observed that both the polyol after degradation and the polyol after rinsing with water exhibit a characteristic peak for C=O stretching vibration in the range of 1700 cm⁻¹ to 1750 cm⁻¹, an amide absorption band in the range of 1610 cm⁻¹ to 1640 cm⁻¹, and a characteristic N-H bending vibration peak in the range of 1540 cm⁻¹ to 1570 cm⁻¹. It indicates that the alcoholamine compound participates in the degradation reaction. The polyol product obtained by degrading the polyurethane material retains a significant amount of urea structure, and the structure thereof exhibits a water soluble property.

### <Polyol Functionality Test>

In order to synthesize the polyurethane material using the polyol obtained after degradation, it is necessary to determine the ratio between the polyol and isocyanate. However, the polyol obtained after degradation has an unknown functionality, so a test for functionality of the polyol is needed. Specifically, the functionalities of the polyols obtained after degradations in Example 1 to Example 4 are assumed to be several values, and then the polyols after degradations are reacted with methylene diphenyl diisocyanate in appropriate proportions. The functionality ranges are determined based on the appearance and physical properties of the synthesized products, so as to obtain a close value of functionality of the polyol product obtained through the method for self-catalytic directional degradation of polyurethane material according to the present disclosure, which is favorable for subsequent polyurethane material synthesis.

However, the isocyanate used in determining the functionality range of the polyol obtained by the method for self-catalytic directional degradation of polyurethane material according to the present disclosure is not limited to methylene diphenyl diisocyanate as used in Example 1 to Example 4. Aromatic diisocyanate, aliphatic diisocyanate, an aromatic diisocyanate derivative, an aliphatic diisocyanate derivative, polymethylene polyphenyl isocyanate, or a mixture thereof can also be used for the synthesis reaction. The assumed functionality values and the close functionality values for the polyols obtained after degradations in Example 1 to Example 4 are shown in the following Table 1.

| Table 1 | | |
|---|---|---|
| | Assumed Functionality Value | Close Functionality Value |
| Example 1 | 2.5, 2.7, 3.0 | 3.0 |
| Example 2 | 2.3, 2.5, 2.7, 3.0 | 2.5 |
| Example 3 | 2.7, 3.0, 3.2, 3.5 | 3.5 |
| Example 4 | 2.7, 3.0, 3.2, 3.5 | 3.2 |

In summary, the present disclosure uses the alcoholamine compound with an amino group carrying an active hydrogen as the degradation solution and controls the heating temperature to degrade the polyurethane material. It offers advantages such as fast degradation, low temperature, high recycling efficiency, and no need for catalysts. The polyol obtained after degradation can be used directly as a raw material for subsequent polyurethane synthesis without the need for rinsing or purification. It enables the polyurethane material to be recycled, eliminating the need for high-energy, complicated purification processes, which makes the material as an environmentally friendly material.

Although the present disclosure has been disclosed with reference to the aforementioned embodiments, the present disclosure is not limited thereto. Anyone skilled in the art can make various changes and modifications without departing from the spirit and scope of the present disclosure. Therefore, the scope of the present disclosure should be determined by the scope of the following claims.

## Claims

1. A method for self-catalytic directional degradation of polyurethane material, **characterized in** comprising:
performing a crushing step, wherein the polyurethane material is crushed to form several polyurethane particles;
performing a mixing step, wherein the several polyurethane particles are mixed with an alcoholamine compound to form a degradation system, and the alcoholamine compound has a structure represented by formula (I) or formula (II): 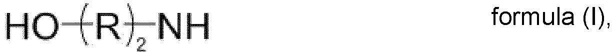 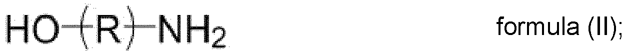
wherein R is an alkylene group having 2 to 6 carbon atoms or a structural isomer thereof, or a phenyl group or a derivative thereof;
performing a degradation step, wherein the degradation system is degraded at a reaction temperature to obtain an intermediate product; and
performing a heating and pressure reduction step, wherein the intermediate product is under the reaction temperature and a reaction pressure to remove the alcoholamine compound, so as to obtain a polyol.

2. The method for self-catalytic directional degradation of polyurethane material of claim 1, **characterized in that**, the polyurethane material is obtained through a polymerization reaction, and a monomer for the polymerization reaction comprises an isocyanate component and an isocyanate-reactive component.

3. The method for self-catalytic directional degradation of polyurethane material of claim 2, **characterized in that**, the isocyanate component comprises aromatic diisocyanate, aliphatic diisocyanate, an aromatic diisocyanate derivative, an aliphatic diisocyanate derivative, polymethylene polyphenyl isocyanate, or a mixture thereof.

4. The method for self-catalytic directional degradation of polyurethane material of claim 2, **characterized in that**, the isocyanate-reactive component comprises a polyester polyol, a polyether polyol, a small-molecule multifunctional compound, or a mixture thereof.

5. The method for self-catalytic directional degradation of polyurethane material of claim 1, **characterized in that**, a particle diameter of each of the several polyurethane particles is 2 mm to 5 mm.

6. The method for self-catalytic directional degradation of polyurethane material of claim 1, **characterized in that**, a mass ratio of the several polyurethane particles to the alcoholamine compound is 1:0.9 to 1:3.

7. The method for self-catalytic directional degradation of polyurethane material of claim 1, **characterized in that**, the reaction temperature is 110°C to 160°C.

8. The method for self-catalytic directional degradation of polyurethane material of claim 1, **characterized in that**, in the degradation step, the degradation system is maintained at the reaction temperature for a degradation time, and the degradation time is 30 minutes to 6 hours.

9. The method for self-catalytic directional degradation of polyurethane material of claim 1, **characterized in that**, the reaction pressure is 1 mbar to 1000 mbar.

10. The method for self-catalytic directional degradation of polyurethane material of claim 1, **characterized in that**, a liquefaction ratio of the polyurethane material after degradation is 100%.

11. A polyol, **characterized in that**, the polyol is obtained by the method for self-catalytic directional degradation of polyurethane material of any one of claim 1 to claim 10.

12. The polyol of claim 11, **characterized in that**, the polyol has a structure represented by formula (III):

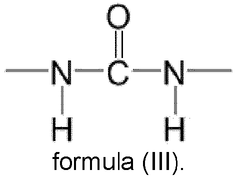

13. A polyurethane material, **characterized in that**, the polyurethane material is obtained by the polyol of claim 11 reacting with isocyanate.
